# EUROPEAN PATENT SPECIFICATION

(11) **EP 2 018 192 B1**
(45) Date of publication and mention of the grant of the patent: **19.09.2012**
(21) Application number: 07732822.7
(22) Date of filing: 15.05.2007
(51) Int. Cl.: C09J 131/04, C09J 139/06, A61L 24/06, A61L 15/58, A61L 24/00

(54) **ADHESIVE SOLUTION FOR APPLICATION TO THE SKIN**
ADHÄSIVE LÖSUNG ZUR APPLIKATION AUF DER HAUT
SOLUTION ADHÉSIVE À APPLIQUER SUR LA PEAU

(30) Priority: 17.05.2006 GB 0609797
(43) Date of publication of application: 28.01.2009
(73) Proprietor: Aston University, Birmingham B4 7ET (GB)
(72) Inventor: AMASS, Alan, Sutton Coldfield B74 3NP (GB); VELON, Maria, Pontevedra, CP 36001 (ES)
(74) Representative: Westwood, Joanna
(86) International application number: PCT/GB2007/001798
(87) International publication number: WO 2007/132239

(56) References cited:
- GB-A- 2 062 663
- US-A- 3 608 070
- US-A- 4 350 785
- US-A- 5 306 504
- US-A1- 2005 281 757

## Description

This invention relates to an adhesive solution for removably fixing items to the skin, especially items that are to be fixed to the skin around a wound or surgically-constructed opening such as a stoma. It is particularly applicable to fixing a colostomy, ileostomy or ureostomy bag having a hydrocolloid flange to the skin so that a stoma can drain into it. Such solutions are applied to the skin and become adhesive as they dry.

Adhesive solutions for the same purpose that are already in use comprise polymer components dissolved in an alcohol such as isopropanol. Other such products are in the form of emulsions rather than solutions.

Adhesive preparations containing a high proportion of alcohol are disliked by patients as they cause stinging when applied around an opening in the skin such as a stoma. Preparations in the form of emulsions are unsatisfactory in that they have limited storage life as the components of the emulsion tend to separate with the passage of time.

Apart from not causing stinging, it is important that an adhesive preparation for removably attaching items to the skin should dry and thus become effectively-adhesive reasonably quickly, preferably within about 60-70 seconds from the time of application.

According to this invention an adhesive solution for removably attaching items to the skin comprises:-
(i) a solids content comprising a vinyl pyrrolidone copolymer, a plasticiser, and a wetting agent;
(ii) water as a first solvent; and
(iii) an alcohol as a second solvent
wherein the amount of alcohol does not exceed 20 wt% of the whole solution, and the glass transition temperature (Tg) of the copolymer composition (i) is below 40°C.

Unless otherwise stated, all percentages are by weight.

Unlike previously-available adhesive solutions, comprising an alcohol such as isopropanol as the only solvent, the adhesive solutions of the invention, being largely water-based and containing only a minor amount of alcohol, do not sting when applied at a wound or surgically-constructed opening such as a stoma. It has also been surprisingly found that such solutions comprising water as the major solvent exhibit similar drying times to compositions comprising isopropanol as the only solvent, although isopropanol has a significantly-lower boiling point (82.4°C) than water. Also, being solutions rather than emulsions, the adhesive solutions of the invention have a longer shelf life than emulsions as they do not tend to separate over time.

The alcohol is preferably isopropanol, although other alcohols such as ethanol and normal propanol may be used. The alcohol should not be of such a high molecular weight that its volatility is insufficient.

The copolymer is preferably a copolymer of PVP and vinyl acetate (PVP/VA). The proportion of vinyl pyrrolidone is preferably from 50-70 wt% and the proportion of vinyl acetate from 50-30 wt%. The most preferred copolymer comprises about 60 wt% of vinyl pyrrolidone and about 40 wt% of vinyl acetate This solution dries to a non-tacky film which is resistant to humidity. A copolymer comprising 70% vinyl pyrrolidone and 30% vinyl acetate is less preferred as it is more brittle (resulting from a higher glass transition temperature) and therefore requires a higher proportion of plasticiser. In addition, it is more soluble in water than a copolymer comprising 60% PVP and 30% VA, which tends to reduce its skin adhesion undesirably.

A variety of physiologically-acceptable plasticisers may be used, and the nature and amount of the plasticiser are selected so that the glass transition temperature of the copolymer composition is less than 40°C, and preferably less than 30°C, and most preferably from 20 to 30°C, e.g. about 22°C. One suitable plasticiser is ethoxylated pentaerythritol (PP150) (15/4EO/OH) which has a molecular weight of 797.

Examples of further possible plasticisers are polyethylene glycols, especially PEG 600 or PEG 200.

Laureth 7 (7, 3, 6, 9, 12, 15, 18, 21-heptoxatritriacontanol) - ethoxylated lauryl alcohol is one example of a compound that acts both as a plasticiser and as a wetting agent.

The adhesive solution may also contain up to about 0.5% by weight of bactericide as a preservative. One suitable preservative is "Germall plus" (trade mark - diazolidinyl urea and idopropynyl butylcarbamate).

The solids content (i) preferably contains at least 75% by weight PVP/VA copolymer and not more than 25% by weight of a combination of plasticiser and wetting agent. Of this combination, about 95% by weight is preferably plasticiser and 5% by weight wetting agent if the proportion of the plasticiser and wetting agent together exceeds 25%, the adhesiveness of the solution after drying will be undesirably decreased. The solids content (i) (i.e. polymer/plasticiser/wetting agent) preferably comprises from 27 to 33% (preferably 30%) of the whole adhesive solution. Of the remainder, about 70% is preferably water and about 10% isopropanol. Drying time increases and adhesiveness decreases if the solids content exceeds about 30%.

This adhesive solution may in addition comprise up to 0.5% of a bactericide as mentioned above and, if necessary, a pH modifier (e.g. sodium hydrogen carbonate) in order to adjust the pH to neutrality.

The adhesive composition is preferably prepared by first adding plasticiser, wetting agent, water and isopropanol in appropriate proportions to a 50% by weight aqueous solution of PVP/VA copolymer. The bactericide (e.g. "Germall plus" in the form of a solid powder) is then added and finally, and if required, a pH modifier, preferably sodium hydrogen carbonate, is added to adjust the pH to neutrality.

One adhesive solution according to the invention will now be described by way of Example only.

### EXAMPLE

A copolymer of vinyl pyrrolidone and vinyl acetate (commercially available under the trade name PVP/VA W635) in the form of a 50% by weight aqueous solution was taken and to it was added the plasticiser PP150 (ethoxylated pentaerythritol) (15/4 EO/OH - molecular weight 797), water, the wetting agent Laureth 7, such that the whole solution comprised 60% water, 10% isopropanol, and 30% solids comprising PVP/PA copolymer plasticiser and wetting agent. Of this the PVP/VA copolymer comprised 75%, and the plasticiser/wetting agent together 25%. Of the latter, the plasticiser comprised 95% and the wetting agent 5%.

To the resulting solution was added the preservative "Germall plus" (up to 0.5% of the whole composition). The pH was then checked by a pH meter and adjusted if necessary to pH 7 by the addition of sodium hydrogen carbonate.

When tested this solution was found not to cause stinging or other irritation when applied to the skin at a wound or around a surgical opening such as a stoma. At room temperature the drying time when applied to the forearm was 78 seconds and when applied to the stomach skin was 60 seconds. The adhesion was measured by impregnating a non-woven cloth (4 x 4 cm) folded double with 0.16ml (0.194g) of solution, and then wiping the impregnated cloth over 8 cm² of skin on the forearm. An average of 0.0237g of the solution remained on the skin. This dried to a thin film. A pressure-sensitive hydrocolloid adhesive material was then applied to the coated area of skin, and left on the skin for 8 hours. After that time it was peeled off and the force/mm required to peel it off was measured, the result being 0.068 N/mm.

Thus the dried solution provided satisfactory adhesion to the skin whilst being easily peeled off when no longer required.

Although the adhesive solution has been described as being particularly suitable for fixing colostomy bags to the skin, it can also be used to fix other items to the skin such as needles or tubes for administering therapeutic solutions by infusion.

It may also be used as an adhesive for bandages or sticking plasters.

## Claims

1. An adhesive solution for removably attaching items to the skin comprising:-
(i) a solids content comprising a vinyl pyrrolidone copolymer, and either a plasticiser, and a separate wetting agent, or a compound that acts as both plasticiser and wetting agent
(ii) water as a first solvent; and
(iii) an alcohol as a second solvent
wherein the amount of alcohol does not exceed 20 wt% of the whole solution, and the glass transition temperature (Tg) of the copolymer composition (i) is below 40°C.

2. An adhesive solution according to claim 1, wherein the copolymer is a copolymer of vinyl pyrrolidone and vinyl acetate.

3. An adhesive solution according to claim 2, wherein the copolymer comprises from 50-70 wt% of vinyl pyrrolidone and 50-30 wt% of vinyl acetate.

4. An adhesive solution according to claim 3, wherein the copolymer comprises about 60 wt% of vinyl pyrrolidone and about 40 wt% of vinyl acetate.

5. An adhesive solution according to any preceding claim, wherein the plasticiser is ethoxylated pentaerythritol or a polyethylene glycol.

6. An adhesive solution according to any preceding claim, wherein the compound that acts both as plasticiser and wetting agent is 7, 3, 6, 9, 12, 15, 18, 21-heptoxatritriacontanol.

7. An adhesive solution according to any preceding claim, wherein the solids content is about from 27 to 33% by weight of the whole solution.

8. An adhesive solution according to claim 7, comprising about 60% water and 10% isopropanol.

9. An adhesive solution according to any preceding claim, wherein the glass transition temperature (Tg) is less than 30°C.

10. An adhesive solution according to claim 9, wherein the glass transition temperature (Tg) is from 20-30°C.

11. An adhesive solution according to any preceding claim, comprising up to 0.5 wt% of a physiologically-acceptable preservative.

12. An adhesive solution according to claim 11, wherein the preservative is a mixture of diazolidinyl urea and idopropynyl butylcarbamate.

13. An adhesive solution according to any preceding claim, having a pH of about 7.

14. An adhesive solution according to claim 13, wherein the pH is adjusted to about 7 by addition of sodium hydrogen carbonate.

15. An adhesive solution according to any of claims 1 to 7 wherein the alcohol is isopropanol.

## Patentansprüche

1. Klebstofflösung für das Festhaften von wiederablösbaren Erzeugnissen auf der Haut, umfassend
(i) einen Feststoffgehalt aus einem Vinylpyrrolidoncopolymer und entweder einem Weichmacher und einem davon getrennten Netzmittel oder einer Verbindung, die sowohl als Weichmacher als auch als Netzmittel dient,
(ii) Wasser als erstes Lösungsmittel und
(iii) einem Alkohol als zweites Lösungsmittel,
wobei die Menge an Alkohol 20 Gew.-% der Gesamtlösung nicht übersteigt, und die Glasumwandlungstemperatur (Tg) der Copolymerzusammensetzung (i) unter 40° C liegt.

2. Klebstofflösung nach Anspruch 1, bei der das Copolymer ein Copolymer aus Vinylpyrrolidon und Vinylacetat ist.

3. Klebstofflösung nach Anspruch 2, bei der das Copolymer 50-70 Gew.-% Vinylpyrrolidon und 50 - 30 Gew.-% Vinylacetat umfasst.

4. Klebstofflösung nach Anspruch 3, bei der das Copolymer ca. 60 Gew.-% Vinylpyrrolidon und ca. 40 Gew.-% Vinylacetat umfasst.

5. Klebstofflösung nach einem der vorhergehenden Ansprüche, bei der der Weichmacher ethoxylierter Pentaerythrit oder ein Polyethylenglycol ist.

6. Klebstofflösung nach einem der vorhergehenden Ansprüche, bei der die Verbindung die sowohl als Weichmacher als auch als Netzmittel dient, 7, 3, 6, 9, 12, 15, 18, 21-Heptoxatritriacontanol ist.

7. Klebstofflösung nach einem der vorhergehenden Ansprüche, bei der der Feststoffgehalt ca. 27 - 33 Gew.-%, bezogen auf das Gesamtgemisch der Gesamtlösung, beträgt.

8. Klebstofflösung nach Anspruch 7, die ca. 60 % Wasser und 10 % Isopropanol umfasst.

9. Klebstofflösung nach einem der vorhergehenden Ansprüche, bei der die Glasumwandlungstemperatur (Tg) unter 30° C liegt.

10. Klebstofflösung nach Anspruch 9, bei der die Glasumwandlungstemperatur (Tg) 20 - 30° C beträgt.

11. Klebstofflösung nach einem der vorhergehenden Ansprüche, die bis zu 0,5 Gew.-% eines physiologisch verträglichen Konservierungsmittels umfasst.

12. Klebstofflösung nach Anspruch 11, bei der das Konservierungsmittel ein Gemisch aus Diazolidinylharnstoff und Iodopropinylbutylcarbamat ist.

13. Klebstofflösung nach einem der vorhergehenden Ansprüche, das einen pH von ca. 7 aufweist.

14. Klebstofflösung nach Anspruch 13, bei der der pH durch Zugabe von Natriumhydrogencarbonat auf ca. 7 eingestellt ist.

15. Klebstofflösung nach einem der Ansprüche 1 bis 7, bei der der Alkohol Isopropanol ist.

## Revendications

1. Solution adhésive pour fixer de façon détachable des articles sur la peau, comprenant :
(i) une teneur en substance solide comprenant un copolymère de vinylpyrrolidone et, soit un plastifiant et un agent mouillant séparé, soit un composé qui sert à la fois de plastifiant et d'agent mouillant ;
(ii) de l'eau en tant que premier solvant ; et
(iii) un alcool en tant que second solvant,
dans laquelle la quantité d'alcool ne dépasse pas 20 % en poids de la solution totale et la température de transition vitreuse (Tg) de la composition de copolymère (i) est inférieure à 40 °C.

2. Solution adhésive selon la revendication 1, dans laquelle le copolymère est un copolymère de vinylpyrrolidone et d'acétate de vinyle.

3. Solution adhésive selon la revendication 2, dans laquelle le copolymère comprend de 50 à 70 % en poids de vinylpyrrolidone et de 50 à 30 % en poids d'acétate de vinyle.

4. Solution adhésive selon la revendication 3, dans laquelle le copolymère comprend environ 60 % en poids de vinylpyrrolidone et environ 40 % en poids d'acétate de vinyle.

5. Solution adhésive selon l'une quelconque des revendications précédentes, dans laquelle le plastifiant est du pentaérythritol éthoxylé ou un polyéthylène glycol.

6. Solution adhésive selon l'une quelconque des revendications précédentes, dans laquelle le composé qui sert à la fois de plastifiant et d'agent mouillant est le 7,3,6,9,12,15,18,21-heptoxatritriacontanol.

7. Solution adhésive selon l'une quelconque des revendications précédentes, dans laquelle la teneur en substance solide constitue de 27 à 33 % en poids de la solution totale.

8. Solution adhésive selon la revendication 7, comprenant environ 60 % d'eau et 10 % d'isopropanol.

9. Solution adhésive selon l'une quelconque des revendications précédentes, dans laquelle la température de transition vitreuse (Tg) est inférieure à 30 °C.

10. Solution adhésive selon la revendication 9, dans laquelle la température de transition vitreuse (Tg) est de 20 à 30 °C.

11. Solution adhésive selon l'une quelconque des revendications précédentes, comprenant jusqu'à 0,5 % en poids d'un conservateur physiologiquement acceptable.

12. Solution adhésive selon la revendication 11, dans laquelle le conservateur est un mélange de diazolidinyl-urée et de butylcarbamate d'idopropynyle.

13. Solution adhésive selon l'une quelconque des revendications précédentes, ayant un pH d'environ 7.

14. Solution adhésive selon la revendication 13, dans laquelle le pH est ajusté à environ 7 par addition d'hydrogénocarbonate de sodium.

15. Solution adhésive selon l'une quelconque des revendications 1 à 7, dans laquelle l'alcool est de l'isopropanol.
